# EUROPEAN PATENT APPLICATION

(11) **EP 2 082 701 A1**
(43) Date of publication of application: **29.07.2009**
(21) Application number: 09151089.1
(22) Date of filing: 22.01.2009
(51) Int. Cl.: A61B 19/00, A61B 17/00, A61B 17/02

(54) **A tool holder**

(30) Priority: 25.01.2008 GB 0801418
(71) Applicant: Prosurgics Limited, High Wycombe Buckinghamshire HP10 9QR (GB)
(72) Inventor: Finlay, Patrick, Beaconsfield, Buckinghamshire HP9 1AE (GB)
(74) Representative: Beattie, Alex Thomas Stewart

(57) **Abstract**

A support arm (1) comprising: a flexible tubular main body (2); and a plurality of beads (3) contained within the tubular main body, wherein in a first configuration a fluid at least partially surrounds the plurality of beads, in a second configuration at least some of the fluid is evacuated such that the rigidity of the arm is increased, and the tubular main body is not substantially fluid-tight such that it is necessary to evacuate at least some of the fluid in the arm throughout a period in which the arm is in the second configuration.

## Description

The present invention relates to a surgical tool supporting device, a method of manufacturing a surgical tool supporting device, and a surgical system.

Endoscopic surgery is known. It is also known to provide a robot which is configured to hold and manoeuvre an endoscope during endoscopic surgery.

During such surgical operations it is often necessary for a tool to be held in a substantially permanent position. Such a tool may, for example, comprise a retractor which is inserted through an incision in a patient and utilised to hold a part of an internal organ of the patient away from the area which is the subject of the operation. One such example of the use of a retractor in this manner is the retraction of a lobe of a liver in bariatric surgery. This role is normally performed by an assistant surgeon holding the tool and performing any minor adjustments in the position of the tool which may be required in order to assist the surgeon.

This approach has drawbacks, however: an assistant surgeon may be required to hold the tool in exactly the same location for several hours; as such the assistant may not hold the tool in a sufficiently static position, the assistant may make an undesired movement of the tool due to miscommunication between the assistant and the surgeon or the assistant may undesirably occupy valuable space within the operating theatre.

The present invention seeks to ameliorate the problems associated with the prior art.

Accordingly an aspect of the present invention provides a support arm comprising: a flexible tubular main body; and a plurality of beads contained within the tubular main body, wherein in a first configuration a fluid at least partially surrounds the plurality of beads, in a second configuration at least some of the fluid is evacuated such that the rigidity of the arm is increased, and the tubular main body is not substantially fluid-tight such that it is necessary to evacuate at least some of the fluid in the arm throughout a period in which the arm is in the second configuration.

Preferably, the device further comprises a tool clamp configured for attachment to the arm and adapted to hold a tool.

Advantageously, the plurality of beads are all of uniform size and shape.

Alternatively, the plurality of beads includes beads of differing sizes.

Alternatively, the plurality of beads includes beads of differing shapes.

Conveniently, the beads are manufactured from polystyrene.

Advantageously, opposing parts of the tubular main body across a diameter thereof are resiliently biased apart.

Alternatively, opposing parts of the tubular main body across a diameter thereof are resiliently biased together.

Advantageously, the device further comprises a valve module adapted to control the evacuation of fluid from the flexible tubular main body.

Preferably, the device is adapted to support more than one type of tool.

Advantageously, the device is adapted to support more than one tool.

Conveniently, the device further comprises a surgical tool supported by the arm.

Typically, the surgical tool is a retractor.

Another aspect of the present invention provides a surgical system including a surgical robot and a surgical tool support device.

Preferably, the surgical robot and surgical tool support device are attached to each other.

Advantageously, the surgical robot is configured to support a first surgical tool and the tool support device is configured to support a second surgical tool.

Another aspect of the present invention provides a valve module for coupling to a surgical tool support device, comprising: a fluid input adapted for coupling to an inner cavity of the tubular main body of the arm; a fluid output adapted for coupling to a conduit; a valve configured to control the fluid communication between the fluid input and fluid output.

Another aspect of the present invention provides a method of manufacturing a support arm, the method comprising the steps of: forming a flexible tubular main body; and providing a plurality of beads within the tubular main body to form the arm, wherein in a first configuration a fluid at least partially surrounds the plurality of beads, in a second configuration at least some of the fluid is evacuated such that the rigidity of the arm is increased, and the tubular main body is not substantially fluid-tight such that it is necessary to evacuate at least some of the fluid in the arm throughout a period in which the arm is in the second configuration.

Conveniently, the method further comprises the step of providing a tool clamp configured for attachment to the arm and adapted to hold a tool.

Advantageously, the step of providing a plurality of beads comprises providing a plurality of beads which are all of uniform size and shape.

Alternatively, the step of providing a plurality of beads comprises providing a plurality of beads including beads of differing sizes.

Alternatively, the step of providing a plurality of beads comprises providing a plurality of beads including beads of differing shapes.

Conveniently, the step of providing a plurality of beads comprises providing a plurality of beads which are manufactured from polystyrene.

Preferably, forming the tubular main body comprises forming a main body in which opposing parts of the tubular main body across a diameter thereof are resilient biased apart.

Advantageously, forming the tubular main body comprises forming a main body in which opposing parts of the tubular main body across a diameter thereof are resilient biased together.

Preferably, the method further comprises the step of providing a valve module adapted to control the evacuation of fluid from the flexible tubular main body.

Another aspect provides a support arm comprising a flexible tubular main body; and a plurality of beads contained within the tubular body, wherein in a first configuration a fluid at least partially surrounds the plurality of beads and in a second configuration at least some of the fluid is evacuated such that the rigidity of the arm is increased.

An embodiment of the present invention will now be described, by way of example, with reference to the accompanying drawings in which:
Figure 1 shows a sectional schematic view of an arm in accordance with an embodiment of the present invention in a first configuration;
Figure 2 shows a sectional schematic view of an arm in accordance with an embodiment of the present invention in a second configuration;
Figure 3 shows an arm in accordance with an embodiment of the present invention being utilised in a surgical operation; and
Figure 4 shows an arm in accordance with an embodiment of the present invention being utilised in a surgical operation in combination with a robot.

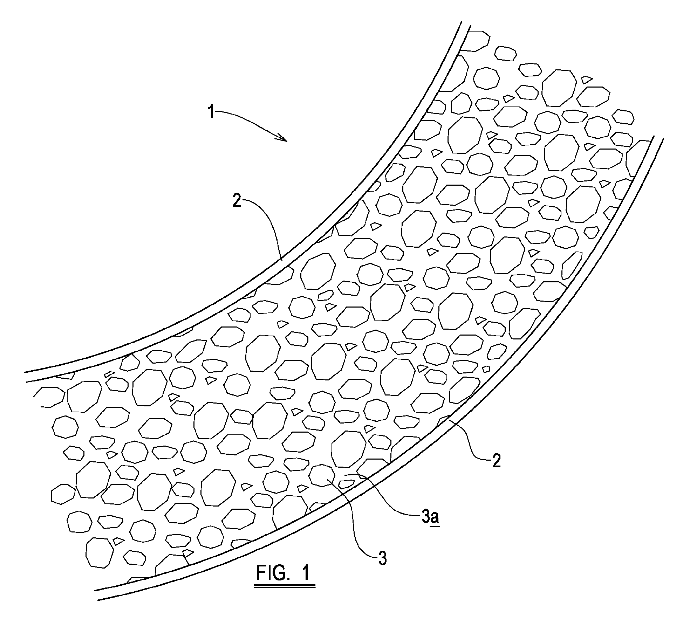

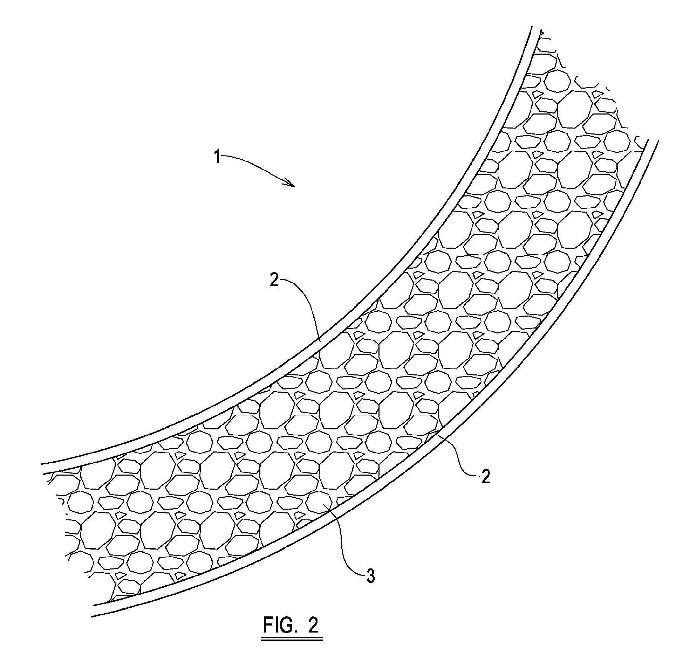

An arm 1 of a surgical tool support device in accordance with an embodiment of the present invention comprises a tubular elongate body having an outer wall 2 defining an inner cavity 3a (see figures 1 and 2).

The tubular elongate body of the arm 1 may have a circular cross-section or may have a different shaped cross-section (e.g. triangular or hexagonal)

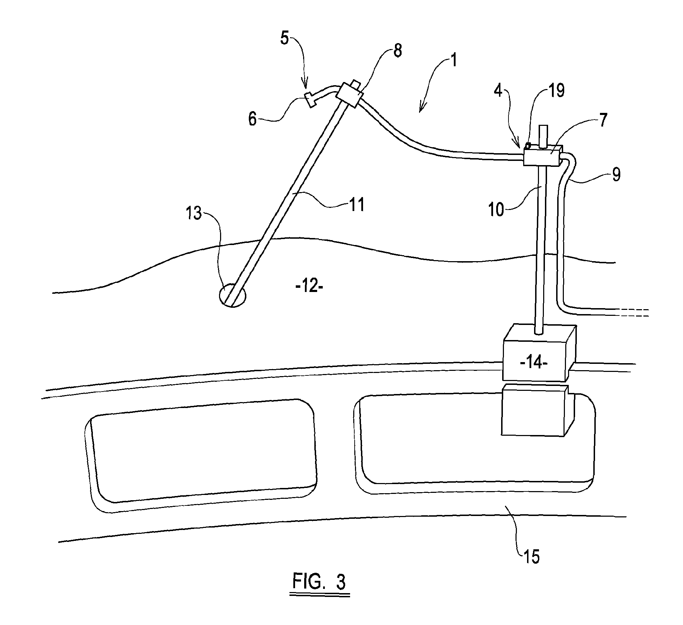

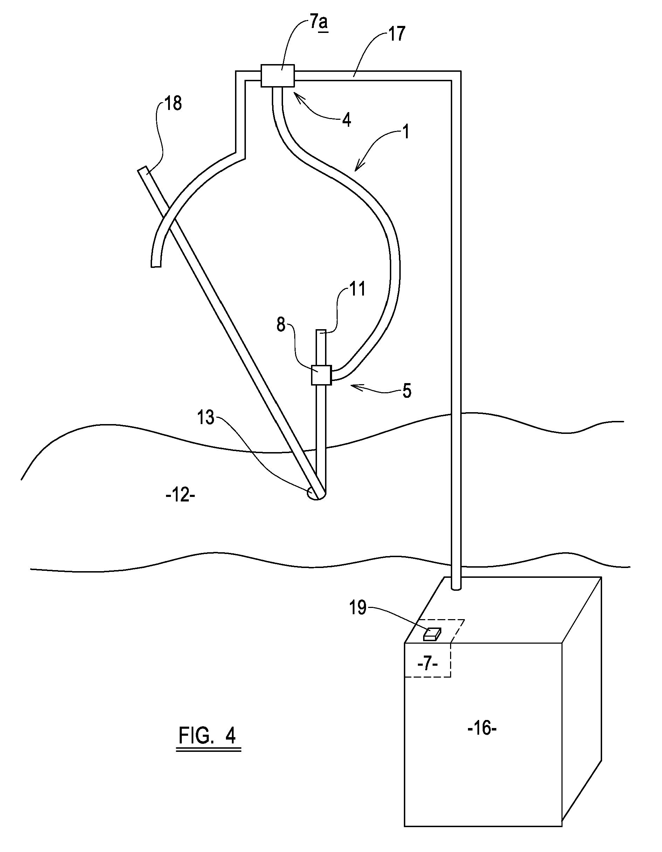

A distal end 5 (see figures 3 and 4) of the arm 1 is substantially sealed. This may be achieved by the use of a plug 6 (as shown in figure 3). The plug 6 may be a separate part which is inserted into the inner cavity 3a of the tubular elongate body of the arm 1 at the distal end 5 thereof. The plug 6 may have a friction fitting, may be adhered to the outer wall 2 of the arm 1, or may be integral with the outer wall 2.

Alternatively, the distal end 5 of the arm 1 may be substantially sealed by adhering opposing parts of the outer wall 2 of the arm 1 together. This may be achieved by using a heated clamp to melt or partially melt the outer wall 2 of the arm 1 such that when the heated clamp is removed opposing parts of the outer wall are adhered to each other.

The outer wall 2 of the arm 1 is constructed from a flexible material which is preferably a polymer material. Conveniently, the outer wall 2 is manufactured out of a material which is substantially fluid-tight (eg. airtight or watertight).

In an embodiment, the outer wall 2 of the arm 1 is resiliently biased into an open configuration. In an embodiment, the outer wall 2 of the arm 1 includes a biasing element (not shown) which biases opposing parts of the outer wall 2 apart (in an open configuration) in order to maximise the size of the inner cavity 3a (for example, a helical spring of resilient material may be embedded in the outer wall 2, or one or more wall separating members may be located in the inner cavity 3a of the arm 1 and configured to bias opposing parts of the outer wall 2 apart).

A proximal end 4 (see figures 3 and 4) of the arm 1 opposes the distal end 5 of the arm 1 across the length of the arm 1. The proximal end 4 of the arm 1 is coupled to a valve module 7. The valve module 7 has a fluid input (not shown) and a fluid output (not shown) which are generally in fluid communication with each other. A valve is located between the fluid input and the fluid output and is configured to control the flow of fluid through the valve module 7 between the fluid input and output thereof. Preferably, the valve is user actuated by, for example, a control switch 19. The control switch 19 may, in fact, be a push-button switch which is directly actuated by the user or may be a switch which is computer controlled.

In an embodiment, the valve may be moved between a first configuration in which the flow of fluid between the input and output is substantially unhindered and a second configuration in which the flow of fluid between the input and output is substantially prevented. Preferably, the valve can be moved to any one of a plurality of positions between the aforementioned first and second configurations.

The proximal end 4 of the arm 1 is coupled to the fluid input of the valve module 7 and may be substantially sealed thereto (such that little or no fluid in the cavity 4 of the arm 1 can escape from the arm 1 except through the fluid input of the valve module 7). In an embodiment, a filter member (not shown) is secured across the entire (or substantially the entire) cross-section of the inner cavity 3a of the arm 1 at the proximal end 4 thereof. The filter member is configured to prevent, substantially, the beads 3 from escaping from the arm 1 but to allow the flow of fluid into and out of the inner cavity 3a of the arm 1. The filter member may form part of the valve module 7.

The fluid output of the valve module 7 is configured to be coupled to a fluid escape conduit 9. This conduit 9 may, for example, be a tube which is attached to a pump or compressor such that the fluid pressure in the tube is less than that of the surrounding atmosphere and/or less than the fluid pressure in the cavity 4 of the arm 1. In an embodiment, the conduit 9 is a vacuum line such as may be found in an operating theatre or laboratory. In an embodiment, the conduit 9 forms part of a support structure 17 for the arm 1 (as shown in figure 4) and the valve module 7 may be located remote from the arm 1 (and may form part of a robot 16 as shown in figure 4).

A second fluid input (not shown) may be provided in the valve module 7. This second fluid input may be in fluid communication with the fluid input of the module 7 (hereinafter, the "first fluid input" for clarity). The valve of the valve module 7 (hereinafter, the "first valve" for clarity) or a second valve of the module 7 may be configured to control the flow of fluid between the first fluid input and the second fluid input in a substantially similar manner to the control over the flow of fluid between the first fluid input and the fluid output.

The second fluid input may be configured to be connected to a source of pressurised fluid or may be vented to the atmosphere.

The second valve may be user actuatable in the same manner as the first valve. A separate control switch (not shown) may be provided for this purpose or the control switch 19 may be adapted for this purpose.

The valve module 7 is adapted so that it may be clamped to a further object. That further object may, for example, be a supporting arm 17 of a robot 16 (as shown in figure 4) or may be a support arm 10 which is secured to a frame 15 by a clamp 14 (as shown in figure 3). In an embodiment, the valve module 7 is adapted to be clamped to one of a plurality of locations along the further object.

A plurality of beads 3 is located in the inner cavity 3a of the arm 1. There is preferably a sufficiently high density of beads 3 in the inner cavity 3a such that in a first configuration (as will be described in more detail below), the arm 1 may be flexed into a desired shape. However, when in a second configuration the arm 1 is substantially rigid.

The beads 3 may be of uniform size and shape; however, the beads 3 are preferably of varying sizes. The shape of the beads 3 may also vary. For example, the beads may have a circular, triangular, hexagonal, or pentagonal cross-section (or, indeed, many other cross-sectional shapes - as will be appreciated). The beads 3 are preferably manufactured from polystyrene. In an embodiment, each bead 3 in an arm 1 is constructed from one of several different materials.

A tool clamp 8 may be secured to the arm 1. In an embodiment, the tool clamp 8 may be secured at any one of a plurality of locations along the length of the arm 1 (as shown in figure 3). In an alternative embodiment, the tool clamp 8 is at a fixed location along a length of the arm 1. This fixed location may be the distal end 4 of the arm 1. Indeed, the tool clamp 8 and the plug 6 may be a single unit.

The tool clamp 8 is configured to secure a tool 11 to the arm 1. The tool 11 may have a maximum cross-sectional diameter of 3 to 12mm and the clamp 8 may be adjustable to hold a tool 11 of this diameter.

In preferred embodiments, the inner cavity 3a of the arm 1 is not substantially fluid-tight. This may be achieved by providing a fluid inlet may be provided in the tool clamp 8 or plug 6. The inlet may be a hole (or plurality of holes) - not shown - in the outer wall 2 of the arm 1. The hole (or plurality of holes) may have a smaller largest diameter than any of the beads 3 (in order to prevent their escape) or may be covered (or substantially covered) by a filter for this purpose.

The operation of embodiments of the present invention will now be described.

The basic operation and purpose of an arm 1 in accordance with an embodiment of the present invention is to provide an arm 1 which can be shaped (ie. flexed) to a desired configuration so as to hold a tool 11 in a substantially fixed location.

This is achieved by providing the arm 1 in a first configuration (as shown in figure 1) in which the beads 3 are not tightly packed within the inner cavity 3a of the arm 1 and the volume around the beads 3 contains a fluid. The arm 1 can then be flexed into the desired configuration. Subsequently, a quantity of the fluid in the inner cavity 3a of the arm 1 is removed (and the arm 1 adopts the second configuration shown in figure 2). This results in the outer wall 2 of the arm 1 reducing in diameter. As the number of beads 3 in the arm 1 has not decreased, the beads 3 are now more closely (ie. densely) packed; this is the second configuration depicted in figure 2. In this configuration, the arm 1 is significantly more rigid than when in the first configuration.

More specifically, an arm 1 in accordance with an embodiment of the present invention may be provided. The arm 1 may be supplied to the user in a blister-pack or other sterile packaging. The arm 1 is preferably a single use item and a fresh arm 1 is utilised for each surgical operation in which such an arm 1 is required.

The arm 1 may be configured such that it may be attached to a valve module 7 at a proximal end 4 of the arm 1 (as shown in figure 3). The arm 1 may also be configured to be attached to an attachment arrangement 7a that forms part of a support structure 17 or robot 16, with the valve module 7 remote from the attachment arrangement 7a (as shown in figure 4). In an embodiment, the arm 1 is supplied with a valve module 7 already attached to the proximal end 4 thereof.

A tool 11 is attached to the arm 1 by the use of a tool clamp 8 which may be located at the distal end 5 of the arm 1 (as shown in figure 4). Alternatively, the tool clamp 8 may be configured to attach to the arm 1 at any one of a plurality of locations along its length (as shown in figure 3).

The tool 11 is positioned in the desired location causing the arm 1 to adopt the desired shape and position. A user may then actuate the valve module 7, preferably by depression of the control switch 19, to cause fluid within the arm to be removed from the hollow cavity 4 of the arm 1. The fluid passes through the fluid input and fluid output of the valve module 7 and is drawn along the fluid escape conduit 9 (due to the lower fluid pressure in the conduit 9 compared to the fluid pressure in the inner cavity 3a of the arm 1). This causes the outer wall 2 of the arm 1 to collapse at least partially to reduce the volume of the inner cavity 3a. As the number of beads 3 within the inner cavity 3a is not substantially reduced, the arm 1 becomes more rigid. If sufficient rigidity is obtained then the tool 11 is substantially held in fixed position.

If the inner cavity 3a of the arm 1 is sufficiently fluid-tight (apart from the fluid inputs and outputs described above), then there may not be a need to evacuate at least part of the fluid in the arm 1 continuously when the arm 1 is in the second configuration. However, it is envisaged that the inner cavity 3a may not be substantially fluid-tight and that, therefore, in order to maintain the desired degree of rigidity of the arm 1 it is necessary to evacuate at least some of the fluid in the arm 1 throughout the period during which the arm 1 is in the second configuration; this may be done continuously or periodically.

If movement of the tool 11 and the arm 1 is required during an operation, then the valve module 7 is actuated to allow fluid to pass into the inner cavity 3a of the arm 1 from the second fluid input to the first fluid input (which now acts as a fluid output). If the inner cavity 3a of the arm 1 is not fluid-tight, then in order to increase the volume of fluid in the inner cavity 3a of the arm 1 it may only be necessary to discontinue any previous continuous or periodic evacuation of the arm 1, in order to allow fluid to enter the inner cavity 3a of the arm 1.

The entry of additional fluid into the inner cavity 3a of the arm 1 causes the volume thereof to increase at least partially. This process reduces the rigidity of the arm 1 and allows the arm 1 to be repositioned. The fluid in the inner cavity 3a can subsequently be at least partially removed (as described above) to increase the rigidity of the arm 1 once again.

The fluid in the arm 1 may be partially or fully evacuated in the above described embodiment to increase the rigidity of the arm 1. Similarly, the quantity of fluid which may be passed back into the arm 1 can, in some embodiments, be controlled to decrease the rigidity of the arm 1. The introduction and evacuation of fluid from the arm 1 may be a continuous-type process by adjustment of the valves of the valve module 7 (in which the quantity of fluid can be varied according to a plurality of different levels between substantially fully evacuated and substantially full inflated) or may be discrete-type process (in which the arm 1 is either substantially fully evacuated or substantially fully inflated) - in other words, the valves of the valve module 7 are either in an open or closed position only.

The fluid may be air, nitrogen, water, saline solution, a gel (ie. a liquid with a higher viscosity than water), or any other fluid which is available to the skilled person and achieves the desired result. It is preferable to use a non-toxic fluid which is also preferably sterile (or substantially sterile) such that, in the event of a leak of the fluid during use, the health of the patient (and surgeons etc) is not put at risk. Preferably, the fluid is air.

The arm 1 may be manufactured in a number of different manners. For example, a polymer tube may be extruded and segmented into lengths (each length will ultimately form an arm 1). Each length of the extruded polymer tube may be clamped in a heated clamp at one end thereof to form a seal. From the open end of the tube a predetermined quantity of beads may be inserted in to an inner cavity defined by the outer wall of the extruded tube. A filter member may then be attached to the open end of the tube. Thus, an arm 1 is formed. In an embodiment, the valve module 7 is attached to the open end of the tube at this stage.

Each arm 1 may be 35cm in length.

It will be appreciated that the arm 1 may be adapted to support more than one tool and/or more than one type of tool. In an embodiment, the arm 1 terminates in a tool holder. Preferably the tool holder can hold at least two different types of tool. Advantageously the tool holder can hold at least two tools at a time.

Other manufacturing methods will be understood by the person skilled in the art to be equally applicable.

Embodiments of the present invention, therefore, provide a simple and inexpensive supporting arm 1 for use in a surgical operation and of particular use in endoscopic surgical operations. There are no complex arrangements of moving parts to allow the arm 1 to lock in position. Thus, the arm 1 can be produced for single use. This avoids the need to sterilise the arm 1 between operations or to drape the arm 1 during operations.

It is not necessary for the inner cavity 3a of the arm 1 to be entirely fluid-tight as a continuous or periodic evacuation of fluid from the arm 1 can be utilised to maintain the rigidity of the arm 1. Thus, expensive sealing and other manufacturing techniques are not required.

Once the arm 1 has been utilised and disposed of, the arm 1 may be at least partially recycled by removal of the beads 3. The beads 3 may be sterilised and reused in a new arm 1 or may be, if made of an appropriate material, melted and moulded into a different product. The polymer tube forming the outer wall 2 of the arm may be melted down and moulded into a different product (if made of an appropriate material).

It will be appreciated that the arm 1 has been described above with the normal state of the arm 1 being flexible (ie. there is sufficient fluid in the arm 1 to allow the arm to be positioned in the desired configuration) and opposing parts of the outer wall 2 being biased apart. However, this is not essential; indeed, the normal state of the arm 1 may be a rigid state with any fluid in the arm 1 being substantially evacuated. Opposing parts of the outer wall 2 may additionally be resiliently biased towards each other. Thus, in order to change the state of the arm 1 to a flexible configuration it is necessary to introduce fluid into the inner cavity 3a of the arm 1. Once the arm 1 has been correctly positioned, the fluid can be evacuated. As such, the fluid evacuation conduit 9 described above may be replaced by a fluid supply conduit 9.

In an embodiment, the tool clamp 8 is omitted and the distal end 5 of the arm 1 is, itself, utilised as a tool. To this end, the distal end 5 of the arm 1 may be shaped to form a support surface adapted to retract the whole or part of an internal organ of a patient.

Embodiments of the present invention may be used in combination with a surgical robot 16 (As shown in figure 4). The surgical robot 16 may be configured to hold and/or move a surgical tool 18 which may be, for example, an endoscope or retractor.

Embodiments of the present invention may be utilised in a method for performing surgery comprising the steps of: providing a surgical tool support device including an arm comprising a tubular main body containing a plurality of beads and a fluid which at least partially surrounds the plurality of beads; attaching a surgical tool to the device; positioning the surgical tool by flexing the tubular main body; evacuating a fluid from at least part of the tubular main body such that the rigidity of the arm is increased.

A method as described above may be performed wherein the step of positioning the surgical tool includes the step of position a surgical retractor to retract part of a patient's anatomy.

It will be understood that the embodiments described above are just an example of an implementation of the present invention. It is envisaged that embodiments of the invention may be utilised in non-surgical applications.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A support arm comprising:
a flexible tubular main body; and
a plurality of beads contained within the tubular main body, wherein in a first configuration a fluid at least partially surrounds the plurality of beads, in a second configuration at least some of the fluid is evacuated such that the rigidity of the arm is increased, and the tubular main body is not substantially fluid-tight such that it is necessary to evacuate at least some of the fluid in the arm throughout a period in which the arm is in the second configuration.

2. An arm according to claim 1, wherein the plurality of beads are all of uniform size and shape, or the plurality of beads includes beads of differing sizes, or wherein the plurality of beads includes beads of differing sizes and the plurality of beads includes beads of differing shapes, or wherein the plurality of beads includes beads of differing shapes.

3. An arm according to any preceding claim, wherein opposing parts of the tubular main body across a diameter thereof are resiliently biased apart or opposing parts of the tubular main body across a diameter thereof are resiliently biased together.

4. An arm according to any preceding claim, adapted to support more than one type of tool and/or adapted to support more than one tool.

5. A surgical tool support device including an arm according to any preceding claims.

6. A device according to claim 5, further comprising a tool clamp configured for attachment to the arm and adapted to hold a tool.

7. A device according to claim 5 or 6, further comprising a surgical tool supported by the arm.

8. A surgical system including a surgical robot and a surgical tool support device according to any one of claims 5 to 7.

9. A system according to claim 8, wherein the surgical robot and surgical tool support device are attached to each other.

10. A system according to claim 8 or 9, wherein the surgical robot is configured to support a first surgical tool and the tool support device is configured to support a second surgical tool.

11. A method of manufacturing a support arm, the method comprising the steps of:
forming a flexible tubular main body; and
providing a plurality of beads within the tubular main body to form the arm, wherein in a first configuration a fluid at least partially surrounds the plurality of beads, in a second configuration at least some of the fluid is evacuated such that the rigidity of the arm is increased, and the tubular main body is not substantially fluid-tight such that it is necessary to evacuate at least some of the fluid in the arm throughout a period in which the arm is in the second configuration.

12. A method according to claim 11, wherein the step of providing a plurality of beads comprises providing a plurality of beads which are all of uniform size and shape, or providing a plurality of beads including beads of differing sizes, or providing a plurality of beads including beads of differing sizes and shapes, or providing a plurality of beads including beads of differing shapes.

13. A method according to claim 11 or 12, wherein forming the tubular main body comprises forming a main body in which opposing parts of the tubular main body across a diameter thereof are resiliently biased apart, or forming a main body in which opposing parts of the tubular main body across a diameter thereof are resiliently biased together.

14. A method of manufacturing a surgical tool supporting device including an arm the method comprising manufacturing an arm according to any one of claims 11 - 13.

15. A method according to claim 14, further comprising the step of providing a tool clamp configured for attachment to the arm and adapted to hold a tool.
